# EUROPEAN PATENT APPLICATION

(11) **EP 3 263 056 A1**
(43) Date of publication of application: **03.01.2018**
(21) Application number: 16181387.8
(22) Date of filing: 27.07.2016
(51) Int. Cl.: A61B 17/34, A61B 5/00

(54) **DEVICE FOR IMPLANTATION OF MEDICAL DEVICES**

(30) Priority: 29.06.2016 US 201662355913 P
(71) Applicant: BIOTRONIK SE & Co. KG, 12359 Berlin (DE)
(72) Inventor: von Arx, Jeffrey, Lake Oswego, OR Oregon 97035 (US); Suwito, Wantjinarjo, West Linn, OR Oregon 97068 (US); Grauhan, Ole, 14163 Berlin (DE)
(74) Representative: Galander, Marcus

(57) **Abstract**

Disclosed is a tunneling-implantation tool 1 used to carry an implantable medical device ("IMD") 2 through an incision and implant the IMD 2 therein. The system can include an IMD 2, an IMD-carrier 4, and a carriage 3. The carriage 3 may be configured to slidably receive the IMD-carrier 4 and assist with deployment of the IMD 3, as well as prevent abrasion of a surface of the IMD 3. The IMD 3 may include a flexible lead 25 comprising an electrode 26 extending from a housing, wherein a distal end 27 of the electrode 25 can exhibit a bullet nose shape. The IMD 2can be mounted on the IMD-carrier 4 and both the IMD 2 and IMD-carrier 4 may be slidably received by the carriage 3 to form a loaded state. Inserting the tunneling-implantation tool 1 through an incision while in the loaded state can cause the bullet nose shaped distal end 27 to spearhead the insertion to create a subcutaneous implant pocket concurrently with the advancement of the IMD 2 through the incision.

## Description

The present invention generally relates to a method for implanting implantable medical devices and a system including a tunneling-implantation tool that may be used with the method, as well as the tunneling-implantation tool itself.

Implantable medical devices ("IMDs") are widely used in the medical field to replace, enhance, or monitor biological structures and biological functions. Some IMDs act autonomously, some work in conjunction with other medical devices, and some function as a hub for several other medical devices. A particular type of IMD is a monitoring device, which may be used to measure and/or sample body signals. Such devices are commonly referred to as implantable monitoring devices. Implantable monitoring devices are typically implanted subcutaneously in a region of the body where they can measure and/or sample relevant body signals. For example, monitoring devices used to measure electrocardiogram ("ECG") signals may be implanted in the upper body region near the heart.

Implantable monitoring devices may be configured to measure and/or sample body signals directly, while others can be configured to measure and/or sample body signals received/transmitted from other apparatuses, implants, and/or sensors. Some of these apparatuses may be external to the body, such as, for example, a patch. The body signals measured from implantable monitoring devices may be measured continuously in real time, sampled from the signal, or acquisitioned from stored data representative of the signal.

IMDs can include a rigid housing containing sensors and other circuitry, or at least facilitating the incorporation thereof. Such devices range in size and shape. Larger sized IMDs, such as, for example, pacemakers and defibrillators, may be inserted by hand without use of a special implant tool; but doing so may be cumbersome and inconvenient. Experiences of the implantation of large sized IMDs show, that for example due to cosmetic reasons a smaller IMD size is favorable, because these devices require only minimal incision sizes, which in turn minimizes the risk of infection and is cosmetically more appealing. But the difficulty of implanting smaller IMDs without a special implant tool increases due to their size and shape and the nature of the implant procedure. Moreover, implantation of IMDs that have flexible housings or external member may become problematic if performed without use of a special implant tool. Further, care should be exercised to not subject an IMD to abrasion during implantation and, in particular, to abrasion from contacting the implant tool.

Prior art methods for IMDs generally consist of deploying the IMD by pushing it with a syringe/plunger-style tool to imbed it within the body of the human or animal. With the syringe/plunger-style tool, an IMD is loaded into a cylinder-like holder and pushed forward by a piston actuated by a plunger until it is imbedded subcutaneously into the body. Such prior art implant methods and tools suffer from many disadvantages that the presently disclosed tunneling-implantation tool and method of use do not.

In particular, prior art implant methods and implant tools often fail to accommodate desired configurations of the IMD, which may include IMDs having extraordinary shapes and IMDs that are flexible or semi-flexible. A flexible or semi-flexible implantable medical device can enable better manipulation and positioning of the device for better sensing capabilities. Yet, prior art implant methods hardly accommodate such IMD features, and prior art implant tools fail to provide an effective means to facilitate their implantation. A particular disadvantage exhibited by the prior art is that while implanting flexible or semi-flexible implantable medical devices, the prior art methods and tools fail to maintain a low profile shape of the implantable medical devices when being inserted through the incision and into the subcutaneous implant pocket of the body. For example, prior art methods and tools for implantation do not prevent the IMD from bending, buckling, or becoming misaligned or askew with the desired orientation when being implanted.

Prior art methods and tools for implantation also often fail to provide a means to create an appropriate sized subcutaneous implant pocket for the IMD by ensuring that the subcutaneous implant pocket is large enough to accommodate the IMD, yet not excessively large.

Prior art methods and tools for implantation also often fail to ensure that the process of implantation does not inadvertently enlarge or cause abrasion to the incision site.

Prior art methods and tools for implantation also often fail to provide a means to ensure that the process of implantation does not cause abrasion to the IMD itself.

Prior art methods and tools for implantation also often fail to provide a means to ensure that creation of the subcutaneous implant pocket and/or subsequent implantation of the IMD does not cause abrasion to the muscle or other tissue.

Prior art methods and tools for implantation also often fail provide a means to ensure that the IMD is not inadvertently implanted into the underlying muscle or other tissue at a non-desired location.

Prior art methods and tools for implantation also often fail to facilitate proper alignment and fail to provide adequate support for the IMD.

Prior art methods and tools for implantation also often fail to provide a combined tunneling and implantation tool, simplifying the implantation procedure.

The present invention is directed toward overcoming one or more of the above-identified problems.

The presently disclosed system includes a tunneling-implantation tool that may be used to carry an implantable medical device ("IMD") through an incision made into a surface of the skin of a being and implant the IMD therein. The system can include three main components, which may be an IMD, an IMD-carrier, and a carriage. The IMD-carrier can be configured to temporarily retain the IMD. The carriage may be configured to slidably receive the IMD-carrier and assist with deployment of the IMD, as well as prevent abrasion of a surface of the IMD. This can include preventing abrasion of a coating (e.g., insulating coating) of the IMD. The IMD may include an electrode extending from a housing, wherein a distal end of the electrode can exhibit a bullet nose shape. In use, the IMD is releasably mounted on the IMD-carrier and both the IMD and IMD-carrier may be slidbaly received by the carriage to form a loaded state. Inserting the tunneling-implantation tool through an incision while in the loaded state can cause the bullet nose electrode to spearhead the insertion and create a subcutaneous implant pocket concurrently with the advancement of the IMD through the incision.

The carriage can further include a ramp that may cause rotation of a stylet portion of the IMD-carrier when the IMD-carrier is at least partially extracted after the IMD has been positioned within the subcutaneous implant pocket. This rotation can prevent scratching and/or abrasion of the IMD while the IMD-carrier and carriage are removed from the incision site to complete the implantation procedure.

Use of the system can provide a preloaded IMD on a tunneling-implantation tool by creating the loaded state. Further, when in the loaded state, the IMD is not only well-situated for implantation, but the system itself can be used to generate the subcutaneous implant pocket as deployment of the IMD is being conducted. This can significantly simplify the implantation process and improve the outcome of the implantation procedure. For example, the simpler implantation process can reduce the time require to perform the implantation. Further, the formation of a suitable sized subcutaneous implant pocket can reduce the proclivity of injury and trauma to the patient, resulting in faster healing and recovery rates for the patient. The simpler implantation procedure and the use of a single-combined tool can lead to less chance of infection. And the snug fit of the IMD within the self-made subcutaneous implantation pocket can result in better Egrams (*i.e.,* ECG signals).

In an exemplary embodiment, a device for implantation of an IMD into the body of a being can include an IMD-carrier including an elongated structure having an IMD-carrier proximal portion and an IMD-carrier distal portion, wherein the elongated structure may extend along a device longitudinal axis. The IMD-carrier can further include a handle at the IMD-carrier proximal portion, a forward-assist extending from the handle, a stylet at the IMD-carrier distal portion, the stylet positioned to extend distally, preferred additionally or alternatively with at least partially underneath the forward-assist, and a retention channel formed into a portion of the IMD-carrier, the retention channel capable of temporarily retaining the IMD. The device can further include a carriage configured to slidably receive the IMD-carrier so that the carriage and IMD-carrier both extend along the device longitudinal axis, with the carriage further include a ramp. When the IMD is mounted within the retention channel, the stylet may be used to support a portion of the IMD, *e.g.* from underneath, and/or insert into a portion of the IMD, and when the IMD-carrier is at least partially extracted from the carriage, the ramp can cause at least a portion of the IMD-carrier to rotate.

In alternative embodiments, the carriage can include a gripping portion formed on opposite lateral sides thereof, and/or an implant/tunneling stopper to prevent over and under insertion of the IMD. Further, the IMD-carrier can include retention tabs temporarily securing the IMD in place within the retention stopper. Further, when the IMD is mounted within the retention channel and the IMD-carrier is slidably received by the carriage, an IMD longitudinal axis and the device longitudinal axis can be made to be parallel to form a loaded state. Additionally, the stylet can be angled so as to form an angle within a range from 4 to 7 degrees relative to the IMD longitudinal axis.

In another exemplary embodiment, a system for implantation of a device into the body of a being can include an IMD including a housing structure at an IMD proximal portion and having a flexible member, preferably a flexible housing structure or a flexible lead, located at an IMD distal portion, and a stylet tunnel formed in a portion of the flexible member. The system can further include an IMD-carrier, the IMD-carrier including an elongated structure having an IMD-carrier proximal portion and an IMD-carrier distal portion, wherein the elongated structure extends along a device longitudinal axis. The IMD-carrier can further include a handle at the IMD-carrier proximal portion, a forward-assist extending from the handle, a stylet at the IMD-carrier distal portion, with the stylet positioned to extend distally, preferred additionally or alternatively with at least partially underneath the forward-assist, and a retention channel formed into a portion of the IMD-carrier, the retention channel capable of temporarily retaining the IMD. The system can further include a carriage configured to slidably receive the IMD-carrier so that the carriage and IMD-carrier both extend along the device longitudinal axis, where the carriage may further include a ramp. When the IMD is placed within the retention channel, the stylet is received within the stylet tunnel and supports a portion of the IMD, *e.g.* from underneath, and /or transfer force applied at the handle to a distal end of the flexible member so that the flexible member can tunnel through tissue during implantation, and when the IMD-carrier is at least partially extracted from the carriage, the ramp can cause at least a portion of the IMD-carrier to rotate.

In alternative embodiments, the carriage can include a gripping portion formed on opposite sides thereof, and an implant/tunneling stopper to prevent over and under insertion of the IMD. Further, the IMD-carrier can further include retention tabs temporarily securing the IMD in place within the retention channel. The IMD distal end of the flexible member may have a bullet nose shape, and the IMD distal end may be reinforced and includes a bullet nose shape. At least a portion of the IMD may be coated with an insulating material. The stylet tunnel is angled within a range from 4 to 7 degrees relative to the device longitudinal axis. The stylet can act as a stiffing element to prevent the IMD from bending, buckling, and/or becoming misaligned or askew. Further, the stylet can support and/or straighten the flexible member during implantation of the IMD, and the bullet nose shape reinforced IMD distal end can be used to facilitate tunneling to generate a subcutaneous implant pocket. The stylet shall be as thick as possible in order to allow the implanter to push with great force on the bullet shaped electrode so that the bullet shaped nose will tunnel through tissue. The force is translated from the handle to the bullet shaped nose via the stylet.

An exemplary use of the system for implantation of a device into the body of a being can include creating an incision in a skin surface of the body; providing a tunneling-implantation tool comprising an IMD-carrier and a carriage, wherein the IMD-carrier is received within the carriage and includes a stylet formed at a distal portion thereof, wherein in a loaded state, the stylet is received in a stylet tunnel formed in a flexible member, preferably flexible housing structure or a flexible lead, located at an IMD distal portion, wherein a retention channel is formed into a portion of the IMD-carrier and wherein an implantable medical device ("IMD") is mounted or pre-mounted into the retention channel of the tunneling-implantation tool; placing a distal portion of the carriage in close proximity of the incision; inserting a distal portion of the IMD-carrier through the incision and advancing the IMD through the incision, wherein the flexible member has a bullet nose shape at its distal end forming a subcutaneous implant pocket during the inserting and advancing step; gripping the carriage and at least partially extracting the IMD-carrier from the incision and the IMD by at least partially withdrawing the IMD-carrier from the carriage; and removing the IMD-carrier and the carriage from an area of the incision.

In one form, the IMD-carries and stylet are rotated during the extracting step, with the rotation is caused by the IMD-carrier contacting a ramp formed in the carriage. The gripping step includes gripping the carriage at gripping portions formed on opposite lateral sides thereof. Advancing the IMD through the incision can include advancing the IMD and the tunneling-implantation tool until an implant/tunneling stopper formed on the carriage abuts against skin surface of the incision. In a further form, the stylet tunnel is angled within a range from 4 to 7 degrees relative to a longitudinal axis of the IMD device.

While these potential advantages are made possible by technical solutions offered herein, they are not required to be achieved. The presently disclosed system and method can be implemented to achieve technical advantages, whether or not these potential advantages, individually or in combination, are sought or achieved.

Further features, aspects, objects, advantages, and possible applications of the present invention will become apparent from a study of the exemplary embodiments and examples described below, in combination with the Figures, and the appended claims.

The above and other objects, aspects, features, advantages and possible applications of the present invention will be more apparent from the following more particular description thereof, presented in conjunction with the following Figures, in which:
- FIG. 1: shows an exemplary perspective view of a tunneling-implantation tool with the implantable medical device ("IMD") in a loaded state.
- FIG. 2: shows an exemplary IMD distal end having a bullet nose shape.
- FIG. 3: shows an exemplary front-end view of the tunneling-implantation tool with the IMD in the loaded state.
- FIGS. 4A-4B: show a partial view of an exemplary IMD distal portion with the IMD in the loaded state and a partial cross-section view of the exemplary IMD distal portion in the loaded state, respectively.
- FIG.5A-5D: show various views of an exemplary tunneling-implantation tool starting in the loaded state and transitioning therefrom to illustrate an IMD-carrier being partially extracted from a carriage to deploy the IMD.
- FIG. 6: shows a partial top view of a stylet portion of the IMD-carrier and a ramp of the carriage.

The following description is of an embodiment presently contemplated for carrying out the present invention. This description is not to be taken in a limiting sense, but is made merely for the purpose of describing the general principles and features of the present invention. The scope of the present invention should be determined with reference to the claims.

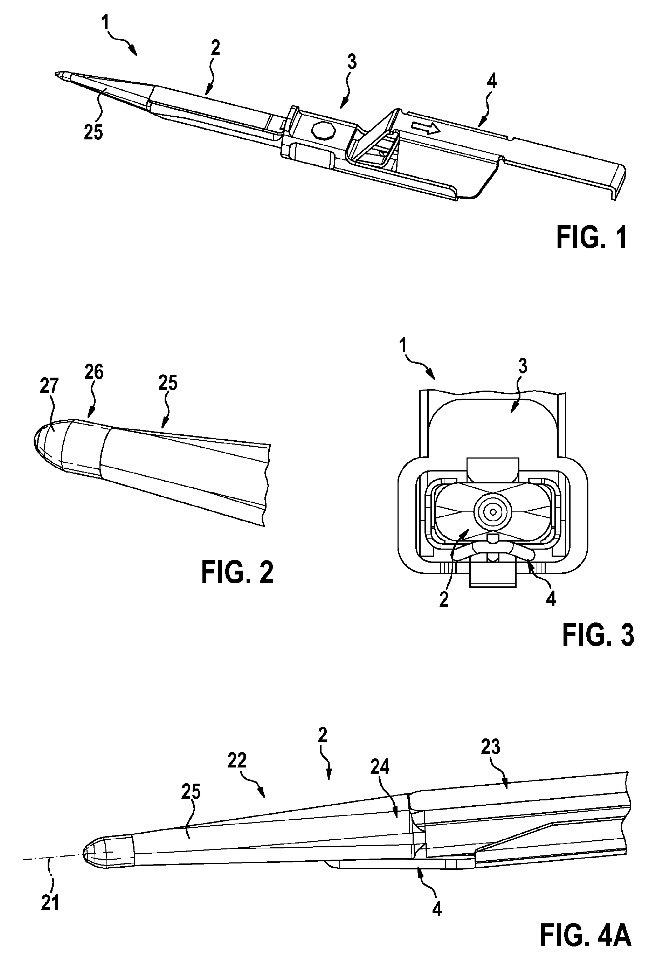

Referring to FIGS. 1-3, the presently disclosed system includes a tunneling-implantation tool 1 that may be used to carry an implantable medical device ("IMD") 2 through an incision made into a surface of the skin of a being. Further, the tunneling-implantation tool 1 may be used to deploy the IMD 2 into a portion of the incision, and thus implant the IMD 2 into the being. In some embodiments, the tunneling-implant tool 1 has an IMD-carrier 4 and a carriage 3. The IMD-carrier 4 can be configured to temporarily retain the IMD 2. The carriage 3 may be configured to slidably receive the IMD-carrier 4 and assist with deployment of the IMD 2, as well as prevent abrasion of a surface of the IMD 2. This can include preventing abrasion of a coating (e.g., via a biomonitor coating) of the IMD 2. The IMD 2 may include flexible lead 25 extending from a housing, wherein at the distal end of the flexible lead 25 an electrode 26 is situated, which can exhibit a bullet nose shaped tip 27 (*see* FIG. 2). In one implementation, the IMD 2 is placed on the IMD-carrier 4 and both the IMD 2 and IMD-carrier 4 are slidably received by the carriage 3 to form a loaded state, as seen in FIG. 1. Portions of the IMD-carrier 4 along with the IMD 2, can then be inserted through an incision with the bullet nose tip 27 of the electrode 25 spearheading the insertion.

As will be described in detail below, features of the tunneling-implantation tool 1 can be utilized to at least partially extract the IMD-carrier 4 from the incision while the implantation-tunneling tool 1 is inserted through the incision to cause deployment of the IMD 2, yet while maintaining the IMD 2 in a desired orientation and without increasing the risk of abrading a surface of the IMD 2. The IMD-carrier 4 can then be removed from the incision while the IMD 2 is left to reside within the incision, thus deploying the IMD 2 for implantation. It is envisioned for the implantation of the IMD 2 to be at a subcutaneous site and, in particular, within a subcutaneous implant pocket formed between a lower surface of the skin and an underlying tissue (e.g., muscle tissue) of the skin. Some embodiments can enable the tunneling-implantation tool 1 to further be used to create the subcutaneous implant pocket. In yet further embodiments, the tunneling-implantation tool 1 can be used to create the subcutaneous implant pocket and concurrently advance the IMD 2 through the incision and into the subcutaneous implant pocket, thus simplifying the implantation process.

The system can include three main components, which may be the IMD2, the IMD-carrier 4, and the carriage 3. When describing any component, a reference to a proximal orientation/position/location shall be an orientation/position/location that is in closer proximity to a user (*e.g.,* a surgeon) than that of a distal orientation/position/location. For example, a distal portion of a component is a portion that is further away from a surgeon when the surgeon is handling and using the component in accordance with the disclosed method as compared to a corresponding proximal portion of the component.

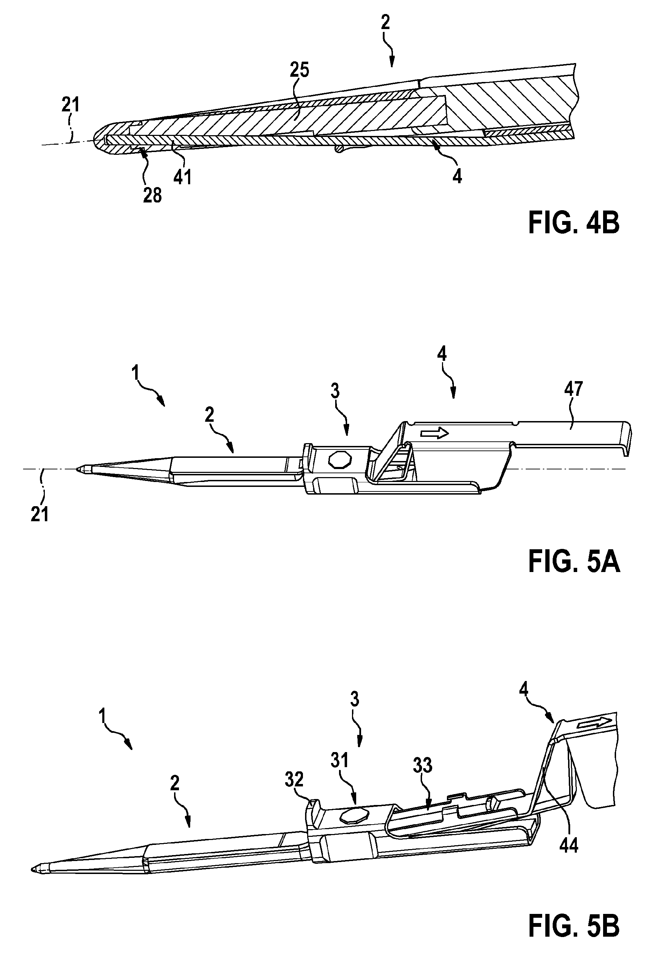

Referring to FIGS. 4A-4B, the IMD can include a housing that may be a unitary housing or may include a plurality of sub-housing structures connected together. Any one of the sub-housing structures can be flexible or rigid. The connection may be barrel hinge, pivot hinge, friction hinge, living hinge, flexure pivot, etc. The flexible nature of some embodiments can enable increased functionality and better conformity with anatomical parts when inserted into a body of a being, and further enable manipulation of the shape of the IMD 2. For example, the housing and/or sub-housings may provide for manual and/or automatic manipulation of the shape to enable contorting the IMD 2 into a desired shape. Some embodiments can include a housing and/or sub-housing that is resilient and/or semi-rigid. Materials used for the housing and/or sub-housing may include polymers, metalalloys, shape-memory material, shape-changing material, softening material, etc..

The housing and/or sub-housing can contain sensors and electrical circuitry, or at least facilitate incorporation thereof, for replacing, enhancing, and/or monitoring biological functions. For example the IMD 2 may include an IMD proximal portion 23 having a first electrically active area connected by the connection to an IMD distal portion 22 having a second electrically active area. The IMD 2 further includes an IMD proximal end and an IMD distal end. In an exemplary embodiment, the IMD proximal portion 23 is a rigid structure and the IMD distal portion 22 is a semi-rigid structure, and they are connected by a flexible connection. The IMD proximal portion 23 and the IMD distal portion 22 may be connected so as to both extend along an IMD longitudinal axis 21, and thus the IMD 2 may be configured as an elongated structure that extends along the IMD longitudinal axis 21. However, any portion of the IMD 2 can be flat, conical, curvilinear, angled, or exhibit other contours and shapes so as to not be aligned along the IMD longitudinal axis 21. The IMD 2 may exhibit rounded edges and corners, which may reduce any tendency to catch on portions of the subcutaneous implant pocket.

The first electrically active area may include a control unit, electrical circuitry, an electrode, etc., which may be configured for telemetric signal transmission. The second electrically active area can be configured as an elongated structure extending from a tapered neck of the IMD distal portion 22, which may include electrical circuitry, a telemetry antenna, an electrode, etc. In one embodiment, the second electrically active area is in a form of a flexible lead 25, wherein at distal end of the flexible lead 25 an electrode 26 is situated as shown in FIG. 4A. A tip 27 of the electrode 25, or the IMD distal end, can exhibit a bullet nose shape or some other tapered shape, spaded shape, frustoconical shape, etc., to assist with tunneling through the incision and generating the subcutaneous implant pocket. This IMD distal end tip 27 can be reinforced to provide added rigidity so as to facilitate tunneling through the incision and separating the skin from the underlying tissue.

The control units and electrical circuitry may include the necessary components to transmit, receive, store, and acquisition analog and digital signals and perform signal processing. This may include, but is not limited to, circuit boards, memory, transmitters, receivers, transceiver circuitry, converters, amplifiers, batteries, etc. The IMD 2 may further include other elements to perform other functions. These may include, but are not limited to, data storage units, sensors, pumps, commutators, etc. Such circuitry and accompanying elements may be rigid, flexible, or semi-flexible as well. For example, any of the electrical elements may include a flexible hardware platform.

In one embodiment, the flexible lead 25 can include a stylet tunnel 28 through which a stylet 41 of the IMD-carrier 4 can be inserted. As will be explained in more detail later, the stylet tunnel 28 can slidably receive a portion of the stylet 41, which may prevent the stylet 41 from making contact with an outer surface of the IMD 2, or at least a portion of the IMD 2. For example, inserting the stylet 41 into the stylet tunnel 28 may be done to prevent the stylet 41 from making contact with an outer surface of the flexible lead portion 25 of the IMD 2 that has been coated. The stylet tunnel 28 is closed when the stylet 41 is not inside the lead. In one embodiment the stylet tunnel 28 contains a silicone disk with a slit in it. The stylet can penetrate the disk through the slit. When the stylet is removed, the slit closes to its relaxed state and provides some measure of a seal.

In at least one embodiment, the IMD 2 can include the necessary circuitry and elements to make it an implantable monitoring device. In addition, the IMD 2 can include a biosensitive coating applied to a surface of any portion of the IMD 2. For instance, the biosensitive coating can be applied to a surface of the IMD distal portion 22. For example, a carbon-based nanotube coating can be applied to a surface of the electrode 26 of the IMD distal end to assist with detecting certain levels of proteins in blood that makes contact with the coating. This is only one example, and other types of biosensitive coating may be used. In another embodiment the body of the IMD is coated with an insulating coating, for example with a poly(p-xylylene) polymer or silicone.

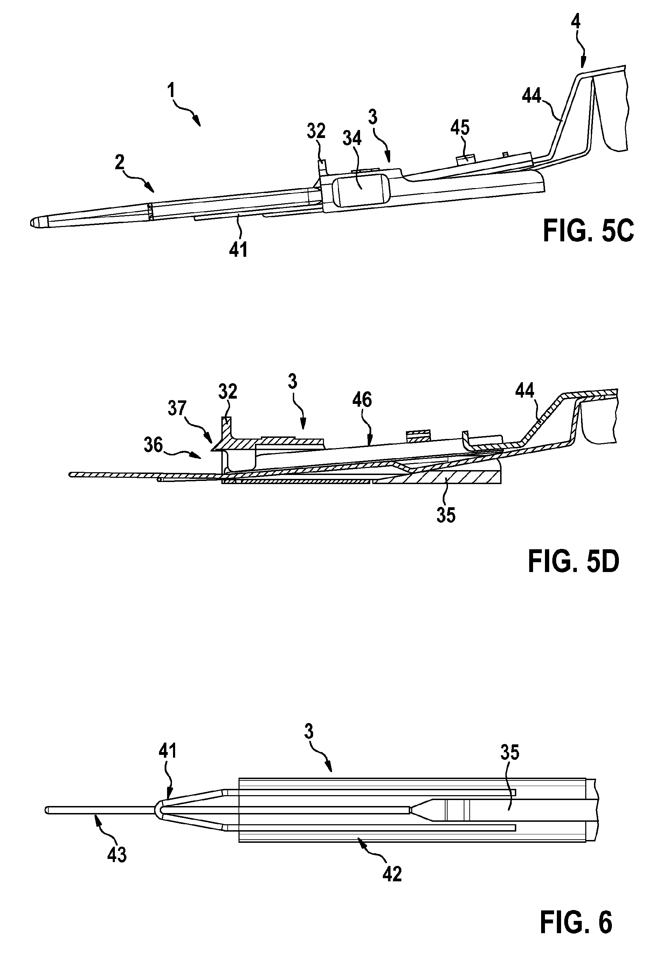

Referring to FIG. 5A, the IMD-carrier 4 can be an elongated structure having a handle 47 at its proximal portion and a stylet 41 at its distal portion. The IMD-carrier 4 may be constructed of a rigid material, such as steel, plastic, aluminum, ceramic, fiberglass, etc. While the IMD-carrier 4 may be rigid, it is envisioned that it may also exhibit some flexibility and resiliency. The IMD-carrier 4 may be configured as an elongated structure that extends along a IMD device longitudinal axis 21. However, any portion of the IMD-carrier 4 can be flat, conical, curvilinear, angled, or exhibit other contours and shapes. In an exemplary embodiment, the IMD-carrier 4 is constructed of steel sheets welded together. The IMD-carrier 4 may further include a retention channel or IMD pocket 46. (*See* FIG. 5D) The retention channel may be a U-shaped channel formed into a portion of the IMD-carrier 4 that is between the handle 47 and the stylet 41. The U-shaped cannel 46 can be constructed from a 0.4 millimeter ("mm") sheet of steel. Other shapes can be used, such as a C-shape, V-shape, etc. During implantation, the IMD 2 can be held within the retention channel 46 while being advanced through the incision. Further, the IMD 2 may be deployed by sliding the IMD 2 from the retention channel 46 and into the subcutaneous pocket. Thus, it is envisioned for the shape of the retention channel 46 to complement the shape of the IMD 2 and, in particular, the shape of the IMD proximal portion 23; however, any shape that provides adequate support and temporary retention of the IMD 2 may be used.

In an exemplary embodiment, the IMD-carrier 4 exhibits an angled structure, having a handle 47 leading to a forward-assist 44. (*See* FIGS. 5C-5D). The handle 47 can be constructed from a 1.0 mm sheet of steel. The angled structure may facilitate easy ingress to and egress from the carriage 3, as well as provide leverage and improve dexterity during use. The angled shape can also enable insertion of the tunneling-implantation tool 1 and deployment of the IMD 2 without abrasion to the incision site or underlying tissue.

The stylet 41 can extend from the handle 47 at a point where the forward-assist 44 extends from the handle 47, and be positioned to extend underneath the forward-assist 44. The stylet may be angled so as to form an angle within a range from 4 to 7 degrees relative to the IMD longitudinal axis 21 when the IMD 2 is placed in the loaded state. This is the angle of the stylet tunnel 28. The stylet 41 can further extend along the device longitudinal axis 21. The stylet 41 can be a rigid and/or semi-rigid (e.g., exhibit some flexibility and resiliency) structure that supports the IMD 2 from underneath or from within (*e.g.*, when inserted into the stylet tunnel 28) when the IMD 2 is placed in the retention channel 46 and during deployment and/or implantation of the IMD 2. In some embodiments, the stylet 41 can be used as a "stiffing" element to prevent the IMD 2 from bending, buckling, and/or becoming misaligned or askew from a desired orientation when being deployed and/or implanted. In other words, the stylet 41 can be used to support and/or straighten the flexible lead 25 during implantation. The stylet 41 may be constructed of a steel, plastic, aluminum, ceramic, fiberglass, etc..

In an exemplary embodiment, the stylet 41 includes an arrangement of first and second stylet structures 42 and 43 (*See* FIG. 6). The first stylet structure 42 can exhibit a picket shape forming a wide base to support the IMD 2. The second stylet structure 43 can be an elongated formation to support the IMD 2, and in particular the flexible lead 25 of the IMD 2. The first and second structures 42 and 43 can be welded together, and the stylet 41 itself can be welded to a main body of the IMD-carrier 4. This complex first and second stylet structure may mirror a transition of the IMD from the flexible lead 25 to a possible wider IMD portion 23. During tunneling the IMD forms his own pocket with support to the tunneling tool, especially with help of the stylet the bullet nose shaped electrode create a tunnel for the lead, then the widening section of the stylet first and second stylet structures widens the tunnel for the wider IMD portion 23 of the implant.

It is contemplated for the stylet 41 to either support the IMD 2 from underneath and/or support the IMD 2 by being inserted within the stylet tunnel 28. The shape and profile of the stylet 41 may be made to complement and accommodate a particular IMD 2. Additionally, the profile of the stylet 41 may be made to accommodate the desired alignment of the IMD 2 upon deployment, tunneling and/or implantation into the subcutaneous implant pocket. As will be evident from the disclosed method of use, the shape and profile of the stylet 41, the "stiffing" feature of the stylet 41, and other features of the implantation-tunneling tool 1, can be used to implant the IMD 2 quickly and effectively.

The carriage 3 can be an elongated semi-tubular structure having a gripping portion 34, a ramp 35, and an implant/tunneling stopper 32. The carriage 3 may be constructed of a rigid material, such as steel, plastic, aluminum, ceramic, fiberglass, etc. As shown in the FIG. 5A, the carriage 3 may be configured as an elongated structure that extends along the device longitudinal axis 21. However, any portion of the carriage 3 can be flat, conical, curvilinear, angled, or exhibit other contours and shapes. The carriage 3 can have a tubular shape, which may be circular, square, triangular, etc. with an open top at its proximal portion to form an IMD-carrier port 33. In an exemplary embodiment, the carriage 3 has a square shape and the structure of the carriage 3 at the IMD-carrier port 33 can be made to form a guide channel 36 for the IMD-carrier 4. The guide channel can include retention tabs or pocket lips 37 (*see* FIG. 5C), which may be used to secure the IMD 2 in place within the guide channel. A bottom surface of the guide channel 36 can include a ramp 35 (*see* FIG. 5D), the ramp 35 being orientated along the device longitudinal axis 21 and having an incline towards the proximal portion of the carriage 3.

A distal portion of the carriage 3 may include an enveloping structure 31, where the gripping portion 34 and the implant/tunneling stopper 32 may be formed on a portion thereof. The gripping portion 34 can include a first grip and a second grip, each disposed on an opposing lateral side of the enveloping structure 31. The gripping portion 34 may be textured or provided with a non-slip coating to provide added dexterity. In an exemplary embodiment, the gripping portion 34 is laser marked to indicate that it is a grip.

The implant/tunneling stopper 32 may be formed on a surface of the enveloping structure 31. As shown in FIGS. 5B-C, the implant/tunneling stopper 32 can be a protrusion extending from the top surface of the enveloping structure 31. However, the implant/tunneling stopper 32 may extend from any surface, and it may extend perpendicularly or at an angle therefrom. The implant/tunneling stopper 32 may be configured to provide added dexterity and support when a user places a finger against the implant/tunneling stopper 32 and the tunneling-implantation tool 1 is being inserted into the incision site. Further, the implant/tunneling stopper 32 may serve as a stopper to prevent over-insertion and/or under-insertion by abutting the skin at the incision when the implant-tunneling tool 1 is inserted into the incision. Allowing the implant/tunneling stopper 32 to about the skin can assure the user that insertion is complete without over-inserting and/or under-inserting.

As described earlier, the ramp 35 can be formed on a bottom surface of the guide channel. The ramp 35 can be constructed from a 0.8 mm sheet of steel. One purpose of the ramp 35 can be to cause the IMD-carrier 4 and/or the stylet 41 to rotate and/or pivot so as to form an angle relative to the IMD longitudinal axis 21 when the IMD-carrier 4 is at least partially extracted during deployment of the IMD 2. Thus, the rotation and/or pivoting can occur automatically as the IMD-carrier 4 is being extracted due to the IMD-carrier's engagement with the ramp 35. This rotation and/or pivoting can prevent scratching or other abrasion to a surface of the IMD 2, and in particular to any coating applied to the IMD 2.

During manufacturing of the system 1 the IMD 2 is put into the loaded state. To create this loaded state the manufacturing of the system 1 (FIGS. 5A - 5D) can include placing the IMD 2 into the retention channel 36 so that the IMD proximal end abuts the forward-assist 44. Placing the IMD 2 into the retention channel 36 can further include sliding the stylet 41 into the stylet tunnel 28. The IMD-carrier 4 can then be slid into the IMD-carrier port 33 of the carriage 3 and through the enveloping structure 31 to form the loaded state, as shown in FIGS. 5A-5B. Alternatively, the IMD-carrier 4 can first be slid into the IMD-carrier port 33 of the carriage 3 and through the enveloping structure 31 before the IMD 2 is placed into the retention channel 36. While in the loaded state, the IMD proximal end can be made to abut the forward-assist 44 so as to prevent the IMD 2 from sliding reward (*i.e.,* toward the proximal portion of the IMD-carrier 4). It may also be possible that the loaded state is performed manually in an operating theatre. This allows the manufacturing of a more customized system 1.

The use of the system 1 can include creating an incision in the skin of a being made with an incision tool (*e.g.,* a scalpel), which can be done before, during, or after the tunneling-implantation tool 1 is placed into the loaded state. The distal portion of the IMD-carriage 3 can then be placed in close proximity of the incision, preferably along a line, which shows the implantation direction. The distal portion of the IMD-carrier 4 may be inserted through the incision with the IMD distal end spearheading the insertion. In an additional or alternative embodiment, a distal portion of the IMD 2 is inserted through the incision. The bullet nose tip 27, the stiffening action of the stylet 41, and the overall shape of the system 1 can enable the system to form a subcutaneous implant pocket as the IMD 2 is advanced through the incision. Thus, creation of the subcutaneous implant pocket can be achieved concurrently with the advancement of the IMD 2 though the incision. The advancement through the incision can continue until the implant/tunneling stopper 32 abuts the skin at the incision site. The simultaneous creation of the subcutaneous implant pocket and the use of the implant/tunneling stopper 32 can be used to ensure that use of the implantation-tunneling tool 1 creates an appropriate sized subcutaneous implant pocket without inadvertent abrasion and/or other injury to the incision site or underlying tissue. Ensuring that an appropriate sized subcutaneous pocket is formed and preventing inadvertent abrasion can thus minimize scaring and reduce risks of infection. Further, the implant/tunneling stopper 32 can be used to ensure that the IMD 2 is not over-inserted and/or under-inserted into the being. For example, if the advancement is too far, damage can occur to the underlying tissue. If the advancement is not far enough, additional maneuvering may have to be performed, leading to undesired trauma. Preventing over-insertion and/or under-insertion can be attributed to the fact that the IMD 2 is carried along with the implantation-tunneling tool 1 during tunneling and implantation and to the fact that the IMD 2 is only inserted as far as the implant/tunneling stopper 32 will allow.

Once fully inserted, a user can grasp the gripping portion 34 to stabilize the carriage 3. The user can then at least partially extract the IMD-carrier 4 by grasping the handle 47 and withdrawing the IMD-carrier 4 from the IMD-carrier port 33 while the carriage 3 is3 held in a stationary position. (*See* FIGS. 5C-5D). Extracting the IMD-carrier 4 can further include retracting the stylet 41 from the stylet tunnel 28, which in one embodiment closes upon retraction of the stylet 41 therefrom.

Withdrawing the IMD-carrier 4 can further cause the IMD 2 to dislodge from the implantation-tunneling tool 1 and remain within the subcutaneous implant pocket. Withdrawing the IMD-carrier 4 can further cause the stylet 41 to engage the ramp and cause the IMD-carrier 4, the stylet 41, and/or other portion of the IMD-carrier 4 to rotate and/or pivot so as to form an angle relative to the IMD longitudinal axis 21so that as the IMD-carrier 4 is being extracted, the stylet 41 does not scratch and/or abrade a surface of the IMD 2. This can include preventing scratching or abrading a portion of the IMD 2 that has been coated. The IMD-carrier 4 can then be fully withdrawn from the carriage 3 via the IMD-port 33, followed by removal of the carriage 3 from the incision site. Alternatively, both of the IMD-carrier 4 and the carriage 3 can be removed from the incision site after the IMD-carrier 4 has been extracted far enough to sufficiently dislodge the IMD 2 so that the IMD 2 has been deployed within the subcutaneous implant pocket even if the IMD-carrier 4 has not been fully withdrawn from the carriage 3. Being deployed can mean that the IMD 2 is dislodged from the tunneling-implantation tool 1 and is seated within the subcutaneous implant pocket so as to further the advancement of the implant procedure. After the IMD 2 has been deployed and the IMD-carrier 4 and carriage 3 have been removed from the incision site, the proximal end of the IMD 2 can be tucked under a skin flap that has been created due to the formation of the subcutaneous implant pocket. The incision can then be closed to complete the implantation of the IMD 2.

As described above, the configuration of the implantation-tunneling tool, along with the method of use described herein, can ensure that deployment of the IMD occurs without inadvertent abrasion and/or other injury to the incision site or underlying tissue, and occurs so as to implant the IMD in a desired orientation within the subcutaneous implant pocket, especially if the IMD is flexible in any manner. The configuration of the implantation-tunneling tool can further enable mounting the IMD thereon such that the IMD longitudinal axis and the device longitudinal axis are coaxial or at least parallel. Therefore, when the implantation-tunneling tool is used to deploy and implant the IMD, a practitioner may be assured that the alignment of the IMD will match that of the implantation-tunneling tool (*i.e.,* the IMD longitudinal axis and the device longitudinal axis are coaxial or at least parallel). At least this feature alone greatly assists the practitioner to ensure proper alignment of the IMD within the subcutaneous implant pocket. Further, the deployment and implantation can be performed without a risk of scratching and/or abrading a surface of the IMD. Additionally, the system, and the method of use, provides for a quick, effective, and easy way to simultaneously create a subcutaneous implant pocket of adequate size and advance the IMD into the subcutaneous implant pocket, thereby significantly simplifying the implantation process.

### Reference List

- 1: Tunneling-implantation tool
- 2: Implantable Medical Device ("IMD")
- 3: Carriage
- 4: IMD-carrier
- 21: IMD longitudinal axis
- 22: IMD distal portion
- 23: IMD proximal portion
- 24: Connection
- 25: Flexible lead
- 26: Electrode
- 27: Distal tip of electrode, Electrode tip
- 28: Stylet tunnel
- 31: Enveloping structure
- 32: Implant/tunneling stopper
- 33: IMD-carrier port
- 34: Gripping portion
- 35: Ramp
- 36: Guiding channel
- 37: Retention tabs or pocket lips
- 41: Stylet
- 42: Stylet first structure
- 43: Stylet second structure
- 44: Forward-assist
- 45: Retention tabs
- 46: Retention channel or IMD pocket
- 47: Handle

## Claims

1. A device for implantation of an implantable medical device ("IMD") (2) into a body of a being, comprising:
an IMD-carrier (4) comprising:
an elongated structure having an IMD-carrier proximal portion and an IMD-carrier distal portion, wherein the elongated structure extends along a device longitudinal axis (21);
a handle (47) at the IMD-carrier proximal portion;
a forward-assist (44) extending from the handle;
a stylet (41) at the IMD-carrier distal portion, the stylet positioned to extend distally the forward-assist; and
a retention channel (46) formed into a portion of the IMD-carrier, the retention channel capable of temporarily retaining the IMD; and
a carriage (3) configured to slidably receive the IMD-carrier so that the carriage and IMD-carrier both extend along the device longitudinal axis, the carriage further comprising a ramp (35),
wherein when the IMD is mounted within the retention channel, the stylet supports a portion of the IMD and/or inserts into a portion of the IMD, and
wherein when the IMD-carrier is at least partially extracted from the carriage, the ramp causes at least a portion of the IMD-carrier to rotate.

2. The device according to claim 1, wherein the carriage (3) further comprises a gripping portion (34) formed on opposite lateral sides thereof

3. The device according to any of the preceding claims, wherein the carriage (3) further comprises an implant/tunneling stopper (32) to prevent over and under insertion of the IMD (2).

4. The device according to claim 1, wherein the IMD-carrier (4) further comprises retention tabs (45) temporarily securing the IMD (2) in place within the retention channel (46).

5. The device according to any of the preceding claims, wherein when the IMD (2) is mounted within the retention channel (46) and the IMD-carrier (4) is slidably received by the carriage (3), an IMD longitudinal axis (21) and the device longitudinal axis are parallel to form a loaded state.

6. The system according to claim 5, wherein when the stylet (41) is received within the IMD (2), the stylet is angled so as to form an angle within a range from 4 to 7 degrees relative to the IMD longitudinal axis (21).

7. A system (1) for implantation of a device into a body of a being, comprising:
an implantable medical device ("IMD") (2), comprising:
a housing structure at an IMD proximal portion (23) and having a flexible member (25) located at an IMD distal portion (22); and
a stylet tunnel (28) formed in a portion of the flexible member;
an IMD-carrier (4) comprising:
an elongated structure having an IMD-carrier proximal portion and an IMD-carrier distal portion, wherein the elongated structure extends along a device longitudinal axis (21);
a handle (47) at the IMD-carrier proximal portion;
a forward-assist (44) extending from the handle;
a stylet (41) at the IMD-carrier distal portion, the stylet positioned to extend distally the forward-assist; and
a retention channel (46) formed into a portion of the IMD-carrier, the retention channel capable of temporarily retaining the IMD; and
a carriage (3) configured to slidably receive the IMD-carrier so that the carriage and IMD-carrier both extend along the device longitudinal axis, the carriage further comprising a ramp (35),
wherein when the IMD is mounted within the retention channel, the stylet supports a portion of the IMD and is received in the stylet tunnel, and
wherein when the IMD-carrier is at least partially extracted from the carriage, the ramp causes at least a portion of the IMD-carrier to rotate.

8. The system according to claim 7, wherein the carriage (3) further comprises a gripping portion (34) formed on opposite lateral sides thereof

9. The system according to claim 7 or 8, wherein the carriage (3) further comprises an implant/tunneling stopper (32) to prevent over and under insertion of the IMD.

10. The system according to any of the claims 7 to 9, wherein the IMD-carrier (4) further comprises retention tabs (45) temporarily securing the IMD in place within the retention channel (46).

11. The system according to claim 7, wherein an IMD distal end (27) of the flexible member (25) has a bullet nose shape.

12. The system according to claim 11, wherein the IMD distal end (27) is reinforced and included a bullet nose shape.

13. The system according to claim 12, wherein the stylet (41) supports and/or straightens the flexible member (25) during implantation of the IMD and the bullet nose shape reinforced IMD distal end (27) facilitates tunneling to generate a subcutaneous implant pocket.

14. The system according to claim 7, wherein at least a portion of the IMD (2) is coated with an insulating material.

15. The system according to claim 7, wherein the stylet tunnel (28) is angled within a range from 4 to 7 degrees relative to the device longitudinal axis (21).

16. The system according to claim 7, wherein the stylet (41) acts as a stiffing element to prevent the IMD (2) from bending, buckling, and/or becoming misaligned or askew.
